Europäisches Patentamt

European Patent Office

Office européen des brevets

(11) Veröffentlichungsnummer: **0 417 038 A1**

## (12) EUROPÄISCHE PATENTANMELDUNG

(21) Anmeldenummer: 90810644.6

(22) Anmeldetag: 27.08.90

(51) Int. Cl.5: **A61B 1/26**

(30) Priorität: 05.09.89 CH 3220/89

(43) Veröffentlichungstag der Anmeldung:
13.03.91 Patentblatt 91/11

(84) Benannte Vertragsstaaten:
DE FR GB NL SE

(71) Anmelder: **Narco-Med AG**
**Hafenstrasse 20**
**CH-8280 Kreuzlingen(CH)**

(72) Erfinder: **Waldvogel, Herman Hans, Dr. med.**
**Bochat 21**
**CH-1094 Paudex(CH)**

(74) Vertreter: **Keller, René, Dr. et al**
**Patentanwälte Dr. René Keller & Partner**
**Postfach 12 Marktgasse 31**
**CH-3000 Bern 7(CH)**

(54) **Laryngoskop.**

(57) Ein Laryngoskop (1) ist mit einer Kraftmeßvorrichtung ausgerüstet, um den zeitlichen Kraftaufwand eines Anaesthesisten während des Freilegens und Freihaltens eines Weges im Mund- und Rachenraum zum Einführen eines Tubus in die Trachea messen und aufzeichnen zu können. Die Kraftmeßvorrichtung hat in der Spatelandruckfläche (17) bzw. in der Nähe des Spatelgelenks angeordnete Druckmeßelemente.

Durch diese Messung läßt sich einerseits objektiv die Fertigkeit des Anaesthesisten beurteilen, andererseits können die für den Intubationsvorgang eingesetzten Pharmaka bezüglich ihrer pharmakologischen Eigenschaften, wie z. B. Hervorrufen einer schnellen und tiefen Muskelrelaxation, objektiv mit Meßwerten belegt werden.

Fig. 1

## LARYNGOSKOP

Die Erfindung betrifft ein Laryngoskop gemäß dem Oberbegriff des Patentanspruchs 1.

Laryngoskope werden insbesondere beim Menschen zum Niederdrücken des Zungengrundes während der Einführung eines Tubus vorbei an den Stimmritzen in die Trachea (Luftröhre) verwendet. Für die Intubation ist eine Lokalanesthesie oder Narkose mit oder ohne Muskelrelaxans notwendig, um eine Hyporeflexie und Muskelrelaxation des Pharynx- und Larynxmuskels zu erzielen.

Ein Laryngoskop dieser Art ist aus der US-PS 4,384,570 bekannt. Das bekannte Laryngoskop hat einen Handgriff, einen abgewinkelt in einen Spatelfuß übergehenden seitensymmetrisch aufgebauten Spatel, der mit seinem Spatelfuß derart gegenüber dem Handgriff verdrehbar ist, daß der Winkel zwischen der Handgriffachse und der Symmetrieebene des Spatels einstellbar ist. An der im klinischen Einsatz den oberen Schneidezähnen des patienten zugewandten Oberseite des Spatels ist eine Druckmeßplatte angeordnet. Die Druckmeßplatte ist mit einer Sicherheitseinrichtung elektrisch verbunden, die ein akustisches oder optisches Signal aussendet, falls ein übermäßiger Anpreßdruck auf die oberen Schneidezähne erfolgt.

Bislang ist es (auch mit dem gemäß US-PS 4,384,570 bekannten Laryngoskop) nicht möglich, objektiv den Intubationsvorgang zu erfassen: es werden deskriptive Kriterien (z. B. schwierige Intubation - leichte Intubation) sowohl für das Anaesthesieprotokoll als auch in der klinischen Erforschung für z. B. neue Muskelrelaxans gebraucht. Aus diesem Grunde ist es für die pharmazeutische Industrie äußerst schwierig wegen unterschiedlicher anatomischer Gegebenheiten der einzelnen Patienten, den unterschiedlichen Handfertigkeiten der ausführenden Anaesthesisten und den oben genannten subjektiven Kriterien pharmazeutische Mittel für Lokalanaesthetica, Muskelrelaxans und Anaesthetika hinsichtlich ihrer pharmakologischen Eigenschaften und klinischen Anwendbarkeit auf ihre Einsatzfähigkeit zu überprüfen.

Der Erfindung liegt die Aufgabe zugrunde, objektive Kriterien und Merkmale einer optimalen Intubation zu schaffen, um einerseits die Ausbildung zukünftiger Anaesthesisten zu vervollkommnen und andererseits objektiv die Wirkung von Lokalanaesthetica, Muskelrelaxans und Anaesthetika ermitteln zu können.

Durch die Erfindung, wie sie in den Patentansprüchen gekennzeichnet ist, wird die obige Aufgabe mit Hilfe eines Laryngoskops gelöst, mit welchem der Kraftaufwand zum Freilegen und Freihalten eines Weges für einen in die Trachea einzuführenden Tubus meßbar ist.

In den Patentansprüchen 2 bis 8 sind bevorzugte Ausführungsarten des erfindungsgemäßen Laryngoskops beschrieben.

Im folgenden werden Beispiele des erfindungsgemäßen Laryngoskops anhand von Zeichnungen näher erläutert. Es zeigen:

Fig. 1 eine Seitenansicht eines erfindungsgemäßen Laryngoskops,

Fig. 2 eine Anwendung des Laryngoskops,

Fig. 3 eine Draufsicht auf die Andruckfläche des Spatels des Laryngoskops,

Fig. 4 eine Vergrößerung eines im Spatel befestigten Druckmeßelements entsprechend des Ausschnitts IV in **Figur 3**,

Fig. 5 einen Schnitt entlang der Linie V - V in **Figur 3**, und

Fig. 6 eine Variante des Laryngoskops.

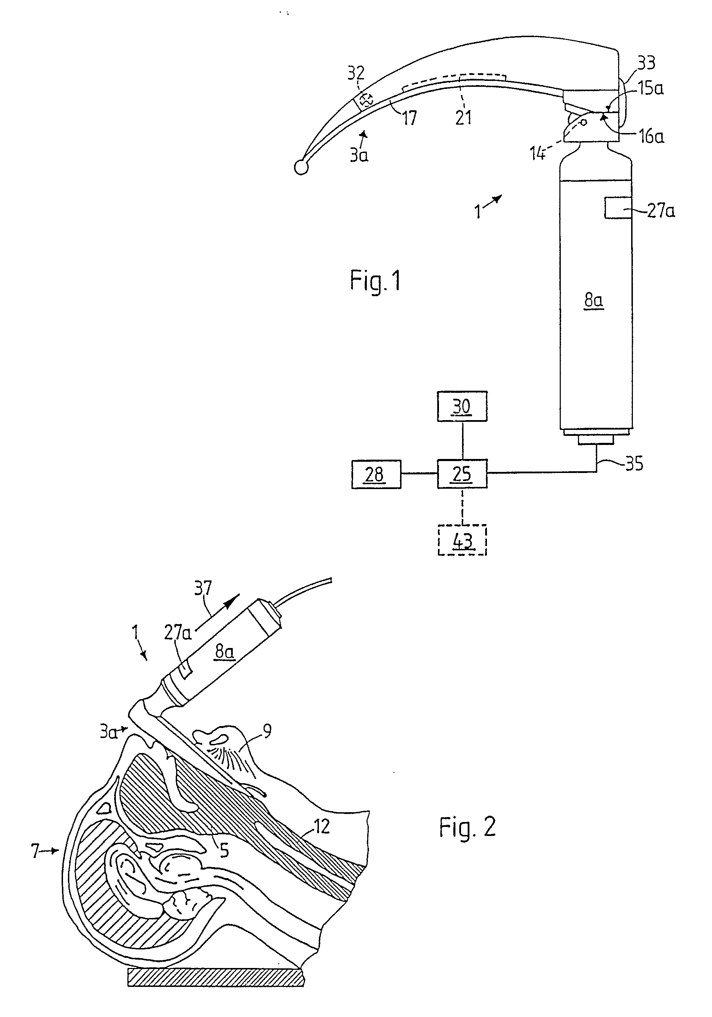

Der aufgeklappte Spatel **3a** des in **Figur 1** dargestellten Laryngoskops **1** ist in den in **Figur 2** dargestellten Mund- und Rachenraum **5** eines Patienten **7** eingeführt. Durch Zug am Handgriff **8a** des Laryngoskops **1** drückt der Anaesthesist den Zungengrund **9** nieder, um den Weg für einen (nicht dargestellten) Tubus an den Stimmritzen vorbei in die Trachea **12** freizulegen und während dessen Einführens freizuhalten.

Der Spatel **3a** ist über ein Gelenk **14** am Handgriff **8a** befestigt und in **Figur 1** annähernd rechtwinkelig zu diesem aufgeklappt, wobei er mit seiner Auslenkungsanschlagflä che **15a** auf der Auslenkungsanschlagfläche **16a** des Handgriffs **8a** an der der Spatelspitze abgewandten Seite aufliegt.

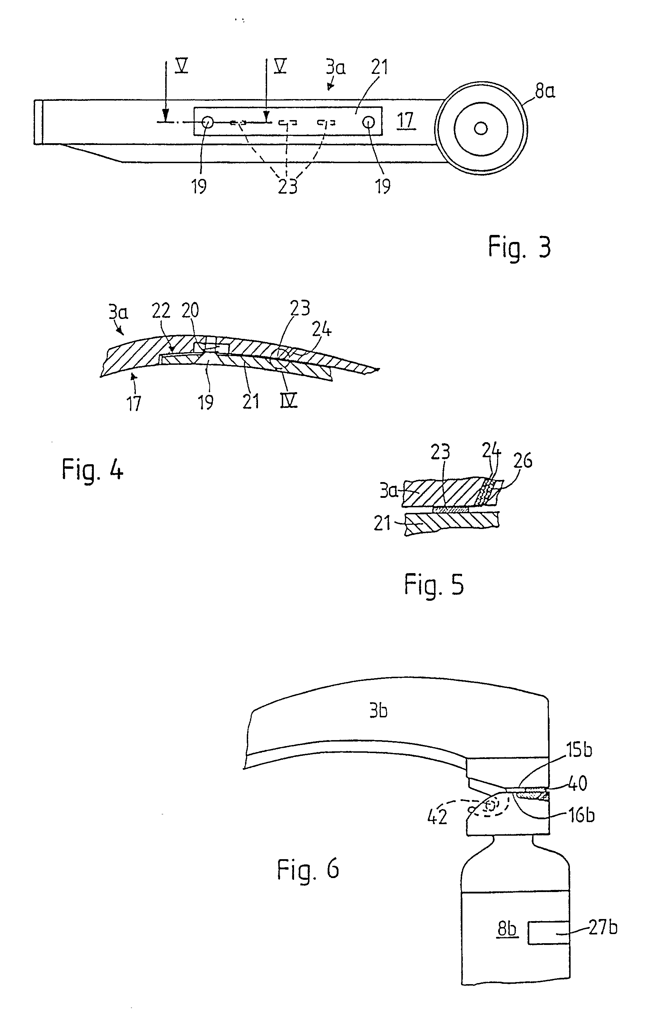

Annähernd in der Mitte der in **Figur 3** dargestellten, konkav gewölbten Andruckfläche **17** des Spatels **3a**, welche zum Freilegen und Freihalten des Weges an den Stimmritzen vorbei in die Trachea **12** dient, liegt eine Andruckplatte **21**, die durch zwei mit je einem Hut versehene Führungen **19** geführt wird, in einer Vertiefung **22** der Andruckfläche **17**, wobei die Hüte ein Herausfallen aus der Andruckfläche verhindern. Die Andruckplatte **21** wird durch zwei Federn **20** leicht gegen die Hüte der Führungen **19** gedrückt; in **Figur 4** sind nur eine Führung **19** und eine Feder **20** dargestellt. In der Vertiefung **22** sind drei piezoelektrische Druckmeßelemente **23** als Meßelemente einer Kraftmeßvorrichtung aufgeklebt. Die elektrischen Zuleitungen **24** zu den Druckmeßelementen **23** sind in einem in **Figur 5** dargestellten Kanal **26**, der zur Oberseite des Spatels **3a** führt, vergossen. Durch die beiden Federn **20** wird die Andruckplatte **21** von der Oberfläche der Druckmeßelemente **23** ferngehalten, um Störinformation zu vermeiden.

Die Kraftmeßvorrichtung besteht aus den bereits erwähnten Druckmeßelementen **23**, einer Auswerteeinheit 25, einer Anzeigeeinrichtung 27 im Handgriff 8a, einer weiteren ortsveränderlichen Anzeigeeinrichtung 28 und einem Schreiber 30 als Aufzeichnungsvorrichtung für die zeitabhängige Aufzeichnung der durch die Auswerteeinrichtung 27 ausgewerteten Meßwerte der Druckmeßelemente 23. Die Stromversorgung für die Kraftmeßvorrichtung und für eine Leuchte 32 im vorderen Drittel des Spatels 3a zur Beleuchtung des Mund- und Rachenraumes 5 während der Intubation ist nicht dargestellt.

Die Leuchte 32 und die Druckmeßelemente 23 sind über ein schematisch in **Figur** 1 dargestelltes, flexibles Kabel 33 mit einem im Handgriff 8a verlaufenden (nicht dargestellten) Kabel verbunden, welches am unteren Ende des Handgriffs 8a mit einem weiteren Kabel 35 zur Auswerteeinrichtung 25 geführt ist.

Zur Durchführung einer Intubation wird der Spatel 3a, wie bereits oben beschrieben, in den Mund- und Rachenraum 5 gesteckt und vom Anaesthesisten mittels des Handgriffs 8a gegen den Zungengrund 9 in Pfeilrichtung 37 in Figur 2 gezogen, wobei die auf den Zungengrund 9 drückende Andruckplatte 21 gegen die Druckelemente 23 gedrückt wird. Die von jedem der drei Druckelemente 23 gemessenen Daten werden von der Auswerteeinrichtung 27 gemittelt, mit einem die Entfernung vom Gelenk 14 berücksichtigenden Wert (Hebelarm) multipliziert und das jeweils in konstanten zeitlichen Abständen errechnete Ergebnis von der Anzeigeeinrichtung 27 angezeigt. Das Ergebnis wird außerdem vom Schreiber 30 zeitabhängig aufgezeichnet.

Diese aufgezeichneten Ergebnisse lassen Rückschlüsse über die "handwerklichen" Fertigkeiten und damit über den Ausbildungsstand des Anaesthesisten zu. Mehrere von Normwerten abweichende zu hohe und/oder zu lang andauernde Kraftaufwendungen lassen auf ungenügende Routine des Anaesthesisten schließen.

Wird zusätzlich zur Zeit-Kraftkurve der Blutdruck des Patienten 7 aufgezeichnet, so kann bei einem stark ansteigendem Blutdruck im Bereich des größten Kraftaufwandes darauf geschlossen werden, daß die Narkosetiefe nicht ausreichend war.

Wird die Wirkung verschiedener Narkotika getestet, so ist der Verlauf der Zeit-Kraftkurve als objektives Vergleichsmittel zu verwenden.

Anstelle die Druckelemente 23 in der Andruckfläche 17 bevorzugt annähernd in der Mitte des Spatels 3a unter der Druckplatte 21 anzuordnen, kann auch, wie in **Figur** 6 darge stellt, ein einziges Druckmeßelement 40 als Kraftmeßelement auf einer Auslenkungsanschlagfläche 16b eines analog zum Handgriff 8a ausgebildeten Handgrlffs 8b angeordnet werden. Die Auslenkungsanschlagfläche

15b eines analog zum Spatel 3a ausgebildeten Spatels 3b drückt dann während des Intubationsvorgangs auf das Druckmeßelement 40. Die vom Druckmeßelement 40 gemessenen Daten werden in der Auswerteeinrichtung mit einem für den Spatel 3b charakteristischen Wert aufgrund dessen Länge und der Lage des Druckmeßelements 40 in eine Kraft umgerechnet, auf einer zur Anzeigeeinrichtung 27a analogen Anzeigeeinrichtung 27b des Handgriffs 8a angezeigt und mit dem Schreiber 30 aufgezeichnet. Als vorteilhaft dieser Anordnung gegenüber dem zuerst beschrieben Laryngoskops ist das fehlende flexible Kabel 33 als Leitung für die Meßwerte der Druckelemente 23, welches u. U. bei wiederholter Sterilisationen des Spatels 3a zu Schwierigkeiten führen könnte, anzusehen. Die Zuleitung zur Lampe 32 wird hier durch (nicht dargestellte) elektrische Kontakte zwischen den Auslenkungsanschlagflächen 15b und 16b ausgeführt. Der Spatel 3b ist im Gegensatz zum Spatel 3a in ein Gelenk 42 am oberen Ende des Handgriffs 8b ein- und aushängbar. Hierdurch kann der Spatel 3b einer äußerst wirkungsvollen Sterilisation nach jeder durchgeführten Intubation unterzogen werden, während der separate Handgriff 8b mit der eingebauten Elektronik schonender sterilisiert wird.

Anstelle die Daten des zeitlichen Kraftaufwands über die separate Auswerteeinrichtung 25 auszuwerten und mit dem Schreiber 30 aufzuzeichnen, können die Daten auch mit einer im Handgriff 8b eingebauten, Auswerteeinrichtung 25 (in **Figur 1** außerhalb des Handgriffes Sa dargestellt) aufgearbeitet werden, welche einerseits den jeweils ermittelten Wert wie oben beschrieben mit der Anzeigeeinrichtung 27b im Handgriff 8b anzeigt und andererseits die zeitabhängigen Daten in einen im Handgriff 8b eingebauten (in **Figur 1** gestrichelt dargestellten) Speicher 43 ablegt, der über ein (nicht dargestelltes) Interface im Handgriff 8b mit einem externen (nicht dargestellten) Lesegerät verbindbar ist. Zur Stromversorgung der elektronischen Baugruppen (Speicher 43, Auswerteeinrichtung 25, Anzeigeeinrichtung 27a bzw. 27b) ist im Handgriff 8a bzw. 8b eine (nicht dargestellte) Batterie eingebaut.

Die im Speicher 43 abgelegten Daten können über das Interface auch direkt von einem (nicht dargestellten) Computer abgerufen und anschließend verarbeitet werden, um z. B. gemittelte Werte, "individuelle Diagramme" der verschiedenen Anaesthesisten, statistische Streuungen, etc. zu ermitteln.

Als Aufzeichnungsvorrichtungen können neben analogen und digital arbeitenden Schreibern 30 auch Bildschirme verwendet werden.

Durch das erfindungsgemäße Laryngoskop ist es erstmals möglich den zeitlichen Kraftaufwand zum Freilegen und Freihalten des Weges für einen in die Trachea einzuführenden Tubus objektiv zu messen. Durch diese Messung läßt sich einerseits die Fertigkeit des Anaesthesisten beurteilen, andererseits können objektiv die für den Intubationsvorgang eingesetzten Pharmaka bezüglich ihrer pharmakologischen Eigenschaften, wie z. B. Hervorrufen einer schnellen und tiefen Muskelrelaxation, mit Meßwerten belegt werden.

**Ansprüche**

1. Laryngoskop **(1)** mit einem Handgriff und **(8a, 8b)** einem an diesem angebrachten Spatel **(3a, 3b)**, mit dem eine Kraftmeßeinrichtung zusammenwirkt, **dadurch gekennzeichnet**, daß die Kraftmeßeinrichtung derart ausgebildet und angeordnet ist, daß der Kraftaufwand zum Freilegen und Freihalten eines Weges für einen in die Trachea **(12)** einzuführenden Tubus meßbar ist.

2. Laryngoskop **(1)** nach Anspruch 1, **dadurch gekennzeichnet**, daß die Kraftmeßeinrichtung eine Auswerte- **(25)** und eine Aufzeichnungseinrichtung **(30)** zur Darstellung und/oder einen Speicher **(43)** zum Abspeichern des zeitabhängig bestimmbaren Kraftaufwands hat.

3. Laryngoskop **(1)** nach Anspruch 1 oder 2, **dadurch gekennzeichnet**, daß die Kraftmeßeinrichtung ein in oder unter der zum Freilegen und Freihalten des Weges für den in die Trachea **(12)** einzuführenden Tubus ausgebildeten Andruckfläche **(17)** des Spatels **(3a)** angeordnetes Druckmeßelement **(23)** hat.

4. Laryngoskop **(1)** nach Anspruch 3, **dadurch gekennzeichnet**, daß der Spatel **(3a)** in seiner Andruckfläche **(17)** mindestens eine bewegliche Platte **(21)** hat, welche die auf sie infolge Freilegens und Freihaltens des Weges für den in die Trachea **(12)** einzuführenden Tubus wirkende Andruckkraft auf das Druckmeßelement **(23)** überträgt.

5. Laryngoskop **(1)** nach Anspruch 2, **gekennzeichnet durch** ein Gelenk **(14, 42)**, welches den Spatel **(3b)** annähernd senkrecht schwenkbar zur Achse des Handgriffs **(8b)** mit diesem verbindet und ein Kraftmeßelement **(40)** der Kraftmeßeinrichtung, welches am Gelenk **(14, 42)** zwischen Spatel **(3b)** und Handgriff **(8b)** angeordnet ist.

6. Laryngoskop **(1)** nach Anspruch 5, **dadurch gekennzeichnet**, daß das Kraftmeßelement **(40)** zwischen einem Auslenkungsanschlag **(15b)** des Spatels **(3b)** am Gelenk **(14, 42)** und dem Handgriff **(8b)** angeordnete ist.

7. Laryngoskop (1) nach Anspruch 6, dadurch gekennzeichnet, daß das Kraftmeßelement ein Druckmeßelement **(40)** ist.

8. Laryngoskop **(1)** nach einem der Ansprüche 2 bis 7, **gekennzeichnet durch** eine im Handgriff **(8a; 8b)** angeordnete, von der Auswerteeinrichtung **(25)** angesteuerte Anzeigeeinrichtung **(27a; 27b)**.

Fig.1

Fig. 2

Fig. 3

Fig. 4

Fig. 5

Fig. 6

6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X,A | US-A-4 384 570 (ROBERTS)<br>* Figuren 2, 5 * * Spalte 2, Zeile 35 - Spalte 3, Zeile 15 *<br>– – – | 1,3,7,8 | A 61 B 1/26 |
| A | US-A-4 295 465 (RACZ ET AL)<br>* Figuren * * Spalte 2, Zeile 38 - Spalte 4, Zeile 54 *<br>– – – – – | 1 | |

RECHERCHIERTE
SACHGEBIETE (Int. Cl.5)

A 61 B
A 61 M

Der vorliegende Recherchenbericht wurde für alle Patentansprüche erstellt

| Recherchenort | Abschlußdatum der Recherche | Prüfer |
|---|---|---|
| Den Haag | 11 Dezember 90 | CHEN A.H. |